(19) Europäisches Patentamt
European Patent Office
Office européen des brevets

(11) **EP 3 881 757 B1**

(12) **EUROPEAN PATENT SPECIFICATION**

(45) Date of publication and mention
of the grant of the patent:
**26.11.2025 Bulletin 2025/48**

(51) International Patent Classification (IPC):
**A61B 5/024** (2006.01)    **A61B 5/1455** (2006.01)
**G16H 50/30** (2018.01)

(21) Application number: **21163304.5**

(22) Date of filing: **18.03.2021**

(52) Cooperative Patent Classification (CPC):
**A61B 5/4854; A61B 5/02416; A61B 5/14551;
A61B 5/7257; G16H 50/30**

(54) **SYSTEM FOR WELLNESS ESTIMATION OF A USER USING PULSE HARMONICS FROM PPG SIGNALS**

SYSTEM ZUR SCHÄTZUNG DES WOHLBEFINDENS EINES BENUTZERS UNTER VERWENDUNG VON PULSHARMONISCHEN AUS PPG-SIGNALEN

SYSTÈME D'ESTIMATION DU BIEN-ÊTRE D'UN UTILISATEUR À L'AIDE D'HARMONIQUES D'IMPULSION À PARTIR DE SIGNAUX PPG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.03.2020 IN 202021012087**

(43) Date of publication of application:
**22.09.2021 Bulletin 2021/38**

(73) Proprietor: **Tata Consultancy Services Limited
Maharashtra (IN)**

(72) Inventors:
• **Ramakrishnan, Ramesh Kumar**
**560066 Banaglore - Karnataka (IN)**
• **Gavas, Rahul Dasharath**
**560066 Banaglore - Karnataka (IN)**
• **Viraraghavan, Venkata Subramanian**
**560066 Banaglore - Karnataka (IN)**
• **Pal, Arpan**
**700160 Kolkata - West Bengal (IN)**
• **PURUSHOTHAMAN, Balamuralidhar**
**560066 Banaglore - Karnataka (IN)**
• **Hissaria, Lalit Kumar**
**560066 Banaglore - Karnataka (IN)**

(74) Representative: **Goddar, Heinz J.
Boehmert & Boehmert
Anwaltspartnerschaft mbB
Pettenkoferstrasse 22
80336 München (DE)**

(56) References cited:
**KR-A- 20120 021 098**

• LU WAN-AN ET AL: "Increased first and second pulse harmonics in Tai Chi Chuan practitioners", vol. 16, no. 1, 29 February 2016 (2016-02-29), XP055829994, Retrieved from the Internet <URL:https://www.researchgate.net/journal/BMC-Complementary-and-Alternative-Medicine-1472-6882/publication/296632023_Increased_first_and_second_pulse_harmonics_in_Tai_Chi_Chuan_practitioners/links/5fc236a1a6fdcc6cc677c445/Increased-first-and-second-pulse-harmonics-in-Tai-Chi-Chuan-practitioners.pdf> DOI: 10.1186/s12906-016-1058-4

• YI-JU SU ET AL: "Power spectral analysis of plethysmographic pulse waveform in pregnant women", JOURNAL OF CLINICAL MONITORING AND COMPUTING, KLUWER ACADEMIC PUBLISHERS, DO, vol. 25, no. 3, 9 August 2011 (2011-08-09), pages 183 - 191, XP019954447, ISSN: 1573-2614, DOI: 10.1007/S10877-011-9291-3

# Description

## TECHNICAL FIELD

The disclosure herein generally relates to wellness monitoring, and, more particularly, to a method and system for wellness estimation of a user.

## BACKGROUND

'Wellness monitoring' broadly refers to an activity of monitoring one or more health parameters and in turn health conditions of users. Depends on purpose/type of wellness monitoring, appropriate methods/systems are used. For example, if a person is being monitored for diagnosis of heart diseases, then suitable sensors for collecting and processing cardiac signals are used.

Photophlethysmogram (PPG) is an optically obtained phlethysmogram that can be used to detect blood volume changes in microvascular bed of tissue. The PPG signals collected can be processed further, so as to perform the health estimation. State of the art systems that use PPG based health estimation approaches exist. However, PPG as a signal has different characteristics/components. As a result, different signal processing approaches can be adopted, each providing varying accuracy and convenience in terms of the signal processing and the health estimation. For example, one of the PPG signal processing approaches that is widely used is Heart Rate Variability (HRV) estimation. HRV consists of changes in time intervals between consecutive heartbeats. The HRV information estimated from PPG signals can be representative of various health conditions of the user. Such methods currently available provide measurement estimates of specific health parameters like heart rate, peripheral capillary oxygen saturation level (SPO2), Heart Rate Variability (HRV) features and not a holistic perspective of wellness. The user has to be well educated on how to interpret these measurements in relation to health and usually requires comparison of data over long periods.

KR 2012 0021098 A discloses a method for evaluating vascular aging using the frequency domain analysis of pulse waves to eliminate an inflection point appearing in a waveform of a pulse wave according to time by analyzing photoplethysmogram in a frequency domain. A pulse wave is detected according to times. The pulse wave is changed to a digital signal. The pulse wave converted to the digital signal is changed to a frequency domain. The pulse wave converted to the frequency domain is standardized. Each power data of peak values according to an age is detected for the peak values of a standardized pulse wave. Regression analysis about the power data is performed. The power data is analyzed in a graph of a function of the age.

## SUMMARY OF THE INVENTION

According to the invention, there is provided a system for estimation of wellness of a user according to claim 1 and a computer program product according to claim 7.

## SUMMARY OF THE DISCLOSURE

Embodiments of the present disclosure present technological improvements as solutions to one or more of the above-mentioned technical problems recognized by the inventors in conventional systems. For example, in one embodiment, a processor implemented method for estimation of wellness of a user is provided. Initially a photoplethysmogram (PPG) signal from the user is collected, over a period of time, via one or more hardware processors. Further, a power spectral analysis of the PPG signal is performed using Fast Fourier Transforms (FFT) to generate a power spectrum of the PPG signal, via the one or more hardware processors. A fundamental frequency (f1) is then estimated as a frequency component with highest magnitude in the power spectrum of the PPG signal, via the one or more hardware processors. Further, 12 harmonics of the fundamental frequency as equal to multiples of the fundamental frequency for 12 iterations are calculated via the one or more hardware processors, and then power of each of the harmonics is calculated via the one or more hardware processors. A plurality of key distinguishing features from the calculated power of each of the 12 harmonics are determined via the one or more hardware processors. Further, a wellness metric is calculated based on the plurality of key distinguishing features, via the one or more hardware processors, wherein the wellness metric represents health condition of the user.

In another aspect, a system for estimation of the wellness of a user is provided. The system includes one or more hardware processors, one or more communication interfaces, and one or more memory (102) storing a plurality of instructions. The plurality of instructions when executed cause the one or more hardware processors to collect a photoplethysmogram (PPG) signal from the user, over a period of time. The system then performs a power spectral analysis of the PPG signal is performed using Fast Fourier Transforms (FFT) to generate a power spectrum of the PPG signal, via the one or more hardware processors. The system further estimates a fundamental frequency (f1) is then estimated as a frequency component with highest magnitude in the power spectrum of the PPG signal, via the one or more hardware processors. Further, 12 harmonics of the fundamental frequency as equal to multiples of the fundamental frequency for 12 iterations are calculated by the system via the one or more hardware processors, and then power of each of the harmonics is calculated via the one or more hardware processors. The system then determines a plurality of key distinguishing features from the calculated power of each of the 12 harmonics via the one or more hardware processors. Further, a wellness metric is calculated based on the plurality of key distinguishing features, via the one or more hardware processors, wherein the wellness metric represents health condition of the user.

In yet another aspect, a non-transitory computer readable medium for estimation of the wellness of a user is provided. The non-transitory computer readable medium initially collects a photoplethysmogram (PPG) signal from the user, over a period of time, via one or more hardware processors. Further, a power spectral analysis of the PPG signal is performed using Fast Fourier Transforms (FFT) to generate a power spectrum of the PPG signal, via the one or more hardware processors. A fundamental frequency (f1) is then estimated as a frequency component with highest magnitude in the power spectrum of the PPG signal, via the one or more hardware processors. Further, 12 harmonics of the fundamental frequency as equal to multiples of the fundamental frequency for 12 iterations are calculated via the one or more hardware processors, and then power of each of the harmonics is calculated via the one or more hardware processors. A plurality of key distinguishing features from the calculated power of each of the harmonics are determined via the one or more hardware processors. Further, a wellness metric is calculated based on the plurality of key distinguishing features, via the one or more hardware processors, wherein the wellness metric represents health condition of the user.

It is to be understood that both the foregoing general description and the following detailed description are exemplary and explanatory only and are not restrictive of the invention, which is defined in the claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for wellness estimation of a user using pulse harmonics extracted from PPG signal of the user, according to some embodiments of the present disclosure.
FIG. 2 is a flow diagram depicting steps involved in the process of the pulse harmonics based health estimation of the user, using the system of FIG. 1, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope of the invention being defined by the following claims.

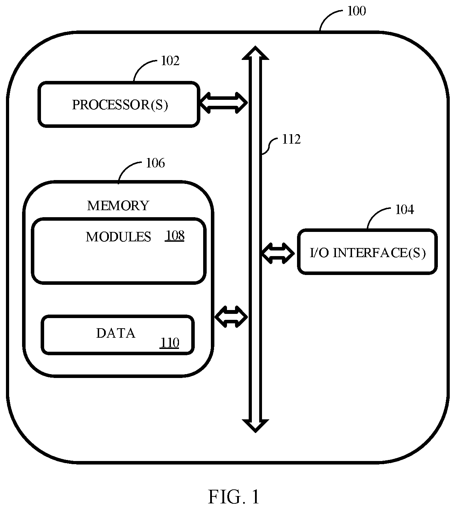

Referring now to the drawings, and more particularly to FIG. 1 through FIG. 2, where similar reference characters denote corresponding features consistently throughout the figures, there are shown preferred embodiments and these embodiments are described in the context of the following exemplary system and/or method.

FIG. 1 illustrates an exemplary system for wellness estimation of a user using pulse harmonics extracted from PPG signal of the user, according to some embodiments of the present disclosure. The system 100 may be implemented in a computing device. Examples of the computing device include, but are not limited to, mainframe computers, workstations, personal computers, desktop computers, minicomputers, servers, multiprocessor systems, laptops, a cellular communicating device, such as a personal digital assistant, a smart phone, and a mobile phone; and the like. The system 100, implemented using the computing device, includes one or more hardware processor(s) 102, IO interface(s) 104, and a memory 106 coupled to the processor 102. The processor 102 can be a single processing unit or a number of units. The hardware processor 102, the memory 106, and the IO interface 104 may be coupled by a system bus such as a system bus 112 or a similar mechanism. The processor 102 may be implemented as one or more microprocessors, microcomputers, microcontrollers, digital signal processors, central processing units, state machines, logic circuitries, and/or any devices that manipulate signals based on operational instructions. Among other capabilities, the processor 102 is configured to fetch and execute computer-readable instructions and data stored in the memory 106.

Functions of the various elements shown in the figures, including any functional blocks labeled as "processor(s)", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non-volatile storage. Other hardware, conventional and/or customized, may also be included.

The IO interfaces 104 may include a variety of software and hardware interfaces, for example, interface for peripheral device(s), such as a keyboard, a mouse, an external memory, and a printer. Further, the IO interfaces 104 may enable the computing device to communicate with other computing devices, such as a personal computer, a laptop, and like.

The memory 106 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. The memory 106 may also include module(s) 108 and data 110.

The modules 108 may include routines, programs, objects, components, data structures, and so on, which perform particular tasks or implement particular abstract data types. The modules 108 may include programs or computer-readable instructions or coded instructions that supplement applications or functions performed by the system 100. The modules 108 may also be used as, signal processor(s), state machine(s), logic circuitries, and/or any other device or component that manipulates signals based on operational instructions. Further, the modules 108 can be used by hardware, by computer-readable instructions executed by the one or more hardware processors 102, or by a combination thereof. In an embodiment, the modules 108 can include various sub-modules (not shown). The other module(s) may include programs or coded instructions that supplement applications and functions of the computing device.

The data 110, amongst other things, serves as a repository for storing data processed, received, and generated by one or more of the module(s) 108. The data 110 includes, for example, history of PPG signals collected over a period of time, wellness metrics calculated for each set of input PPG signals collected, and any such data that are collected or generated during the wellness monitoring being performed by the system 100. The other data includes data generated as a result of the execution of one or more modules in the other module(s).

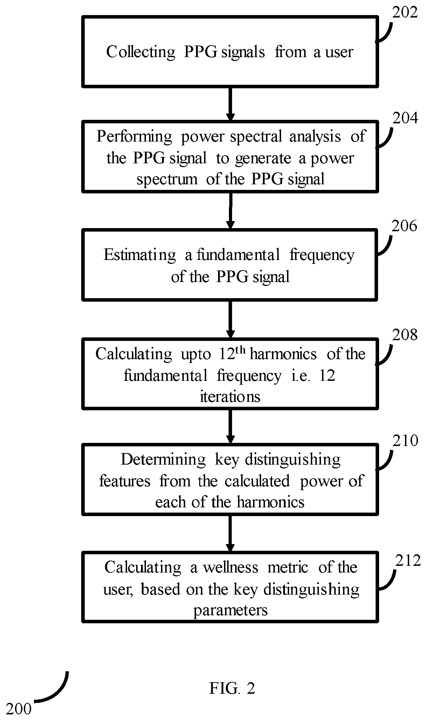

Steps involved in the process of performing the wellness estimation by the system 100 are depicted in FIG. 2, and are explained with reference to the system 100.

FIG. 2 is a flow diagram depicting steps involved in the process of the pulse harmonics based wellness estimation of the user, using the system of FIG. 1, according to some embodiments of the present disclosure. Initially the system 100 collects (202) a PPG signal from a user (who may be a patient being monitored), using any known sensor such as a pulse oximeter. The PPG signal maybe collected for a specific time period as may be required.

Further, the system 100 performs (204) a power spectral analysis of the collected PPG signal. In an embodiment, the system 100 may process the collected PPG signal at once, or may split the PPG signal to segments of specific length and process, as configured. The system may use Fast Fourier Transform (FFT) or any other such suitable approach to perform the power spectral analysis. After performing the power spectral analysis, the system 100 may use direct component of the power spectrum signals for A3 and A4 metrics.

The system 100 further estimates (206) a frequency component with highest magnitude in the power spectrum of the PPG signal, and which is closest to a normal heart rate, as a fundamental frequency ($f1$) of the PPG signal. Here the value of the 'normal heart rate' may be pre-configured with the system 100.

The system 100 further calculates $n^{th}$ harmonics (pulse harmonics) of the fundamental frequency ($f1$) as multiples/iterations of the fundamental frequency ($f1$) i.e.

$$n^{th} \text{ harmonics fn} = n * f1 \text{ --- (1)}$$

where $f1$ is the fundamental frequency, and n = 2, 3, ....., 12

The system 100 further calculates power (pn) of each of the calculated harmonics, and the calculated power is denoted as p2, p3, ... p12. It was observed that peaks are too small to be processed, post $12^{th}$ harmonic. However, it is to be noted that the system 100 can be configured to calculate harmonics and power spectrum past the $12^{th}$ harmonics if required.

Further at step 210, the system 100 determines (210) a plurality of key distinguishing features from the calculated power of each of the harmonics, by computing a separation capability of each of the harmonic powers independently of pulse harmonics p2, p3, ...p12 so as to determine one or more of the features p2, p3, ...p12 as key distinguishing features relating to variations in health condition, which may be result of any fitness activity being performed by the user. The 'separation capability' refers to ability of a feature to consistently distinguish between different states of a ground truth. The ground truth in the case of wellness estimation is a user being monitored being healthy or unhealthy.

Based on the determined key distinguishing features, the system 100 calculates (212) a wellness metric. The wellness metric includes one or more wellness indexes that represent different health conditions of the user being monitored. In order to determine improvement in health condition of the user over a period of time, wellness metrices are calculated at a first time instance T1 and at a second time instance T2. The wellness metric at time T1 and the wellness metric at time T2 are compared to generate a first wellness index (A1), a second wellness index (A2), a third wellness index (A3), and a fourth wellness index (A4).

The first wellness index (A1) of the user is determined as:

$$A_1 = \frac{[\alpha(T1) - \alpha(T2)]}{\alpha(T1)} - (2)$$

where, $\alpha$ = the sum of the powers of the 5th to 12th harmonics from among the 12 iterations, $T_1$ is the first time instance, and $T_2$ is the second time instance.

The first wellness index (A1) represents a general health condition of the user being monitored.

The second wellness index (A2) of the user is determined as:

$$A_2 = \frac{[\alpha(T1) - \alpha(T2)]}{\alpha(T1)} - (2)$$

where, $\alpha$ = the sum of the powers of the 7th , 9th and 10th harmonics from among the 12 iterations, $T_1$ is the first time instance, and $T_2$ is the second time instance.

The second wellness index (A2) represents improvement in health of the user due to one or more fitness activities performed during the time between T1 and T2 a general health condition of the user being monitored.

The third wellness index ($A_3$) is determined as:

$$A3 = \frac{\sum_{i=5}^{12} P_i}{\sum_{i=1}^{12} P_i}$$

where P is power of the $i^{th}$ harmonic.

The third wellness index A3 represents a point in time general wellness of the user.

The fourth wellness index ($A_4$) is determined as:

$$A4 = \frac{\sum_{i=7,9,10} P_i}{\sum_{i=1}^{12} P_i}$$

where P being the power of the $i^{th}$ harmonic.

The fourth wellness index A4 represents a point in time wellness of the user due to effect of the one or more physical activities.

In a practical application, the system 100 can be configured to determine improvement in wellness or health condition of a user, over a period of time, by applying the method 200. In this approach, for a user, the system 100 calculates a first wellness metric at a first time instance T1, and a second wellness metric at a second time instance T2. The system 100 then compares the first wellness metric and the second wellness metric. By virtue of the comparison, the system 100 generates a first wellness index A1 a second wellness index A2. A1 represents improvement in a general wellness of the user, and A2 represents improvement in health of the user, due to one or more physical activities performed by the user during the time period between T1 and T2. For example, consider that the user had been practicing Yoga during this time period. In that case the wellness index A2 represents improvement in health condition of the user due to Yoga.

In an experimental setup, a first group of people (referred to as Yoga Group) containing 36 subjects practiced yoga for an experiment period of 12 days. PPG signals were taken from each of the subjects on first day of the experiment period and on last day (i.e. 12th day) of the experiment period. A second group of people (referred to as a 'control group') containing 24 subjects, with the subjects having different exercise patterns. From the subjects in the control group also, PPG signals were collected on first day and on the 12th day of the experiment period. Table. 1 below contains values of different parameters for the subjects in the first group and the second group.

**Table. 1**

| Pulse Harmonics | | |
|---|---|---|
| Feature | Yoga group | Control group |
| *f1* | 0.22 | 0.31 |
| P1 | 0.37 | 0.26 |
| P2 | 0.16 | 0.57 |
| P3 | 0.65 | 0.29 |
| P4 | 0.16 | 0.54 |
| P5 | 0.01 | 0.47 |
| P6 | 0.03 | 0.45 |
| P7 | 0.01 | 0.84 |
| P8 | 0.01 | 0.54 |
| P9 | 0.0001 | 0.83 |
| P10 | 0.01 | 0.73 |
| P11 | 0.02 | 0.41 |
| P12 | 0.01 | 0.27 |

The values indicate that while there has not been any significant change in the power-values for the subjects in the control group, there is a significant change for the subjects in the yoga group, from P5 to P12, which in turn depicts improvement in wellness/health of the subjects due effect of the physical activity (which in this example is Yoga).

The written description describes the subject matter herein to enable any person skilled in the art to make and use the embodiments. The scope of the subject matter embodiments may include other modifications that occur to those skilled in the art.

The embodiments of present disclosure herein address unresolved problem of wellness estimation using pulse harmonics extracted from a PPG signal of a user being monitored. The embodiment thus provides a PPG based wellness estimation of a user. Moreover, the embodiments herein further provide a mechanism of using the pulse harmonics based wellness estimation to determine improvement in wellness/health condition due to effect of any physical activity being practiced by a user.

It is to be understood that the scope is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of the invention being defined by the following claims.

## Claims

1. A system (100) for estimation of wellness of a user, comprising:

    one or more hardware processors (104);
    one or more communication interfaces (106); and
    one or more memory (102) storing a plurality of instructions, wherein the plurality of instructions when executed cause the one or more hardware processors to:

        collect (202) a photoplethysmogram PPG signal from the user, over a period of time;
        perform (204) a power spectral analysis of the PPG signal using Fast Fourier Transforms FFT to generate a power spectrum of the PPG signal;
        estimate (206) a fundamental frequency f1 as a frequency component with highest magnitude in the power spectrum of the PPG signal, via the one or more hardware processors;
        calculate (208) harmonics of the fundamental frequency as equal to multiples of the fundamental frequency for 12 iterations to generate 12 harmonics;
        calculate power of each of the 12 harmonics;

determine (210) a plurality of key distinguishing features from the calculated power of each of the 12 harmonics; and

calculate (212) a wellness metric based on the plurality of key distinguishing features, wherein the wellness metric represents health condition of the user, wherein the wellness metric is used to determine improvement in the health condition of the user over a period of time, by:

calculating a first wellness metric at a first time instance T1, via the one or more hardware processors;
calculating a second wellness metric at a second time instance T2, via the one or more hardware processors; and
comparing the first wellness metric and the second wellness metric, comprising:

determining a first wellness index A1 of the user, wherein the first wellness index represents a general wellness of the user; and
determining a second wellness index A2 of the user, wherein the second wellness index represents improvement in health of the user due to one or more physical activities performed during the time between T1 and T2.

2. The system (100) as claimed in claim 1, wherein the system computes the plurality of key distinguishing features by calculating a separation capability of the power of each of the harmonics independently.

3. The system as claimed in claim 1, wherein the system determines the first wellness index A1 as:

$$A_1 = \frac{[\alpha\,(T1) - \alpha\,(T2)]}{\alpha\,(T1)}$$

where, $\alpha$ = the sum of the powers of the 5th to 12th harmonics from among the 12 iterations, T1 is the first time instance, and T2 is the second time instance.

4. The system as claimed in claim 1, wherein the system determines the second wellness index A2 as:

$$A_2 = \frac{[\alpha\,(T1) - \alpha\,(T2)]}{\alpha\,(T1)}$$

where, $\alpha$ = the sum of the powers of the 7th, 9th and 10th harmonics from among the 12 iterations, T1 is the first time instance, and T2 is the second time instance.

5. The system as claimed in claim 1, wherein the system determines a third wellness index A3 as:

$$A3 = \frac{\sum_{i=5}^{12} P_i}{\sum_{i=1}^{12} P_i}$$

where P is power of the ith harmonic.

6. The system as claimed in claim 1, wherein the system determines a fourth wellness index A4 as:

$$A4 = \frac{\sum_{i=7,9,10} P_i}{\sum_{i=1}^{12} P_i}$$

where P being the power of the ith harmonic.

7. A computer program product comprising a non-transitory computer readable medium having a computer readable instructions embodied therein, wherein the computer readable instructions, when executed, cause to perform estimation of wellness of a user, by:

collecting a photoplethysmogram PPG signal from the user, over a period of time, via one or more hardware processors;
performing a power spectral analysis of the PPG signal using Fast Fourier Transforms FFT to generate a power spectrum of the PPG signal, via the one or more hardware processors; estimating a fundamental frequency f1 as a frequency component with highest magnitude in the power spectrum of the PPG signal, via the one or more hardware processors;
calculating harmonics of the fundamental frequency as equal to multiples of the fundamental frequency for 12 iterations to generate 12 harmonics, via the one or more hardware processors;
calculating power of each of the 12 harmonics, via the one or more hardware processors;
determining a plurality of key distinguishing features from the calculated power of each of the 12 harmonics, via the one or more hardware processors; and
calculating a wellness metric based on the plurality of key distinguishing features, via the one or more hardware processors, wherein the wellness metric represents health condition of the user, wherein the wellness metric is used to determine improvement in the health condition of the user over a period of time, comprising:

calculating a first wellness metric at a first time instance T1, via the one or more hardware processors;

calculating a second wellness metric at a second time instance T2, via the one or more hardware processors; and

comparing the first wellness metric and the second wellness metric, comprising:

determining a first wellness index A1 of the user, wherein the first wellness index represents a general wellness of the user; and

determining a second wellness index A2 of the user, wherein the second wellness index represents improvement in health of the user due to one or more physical activities performed during the time between T1 and T2.

**Patentansprüche**

1. System (100) zur Schätzung des Wohlbefindens eines Benutzers, umfassend:

einen oder mehrere Hardwareprozessoren (104);

eine oder mehrere Kommunikationsschnittstellen (106); und

einen oder mehrere Speicher (102), die eine Mehrzahl von Anweisungen speichern, wobei die Mehrzahl von Anweisungen, wenn sie ausgeführt werden, den einen oder die mehreren Hardwareprozessoren zu Folgendem veranlassen:

Erfassen (202) eines Photoplethysmogramm-PPG-Signals von dem Benutzer über einen Zeitraum;

Durchführen (204) einer spektralen Leistungsanalyse des PPG-Signals unter Verwendung von schnellen Fourier-Transformationen (Fast Fourier Transforms) FFT, um ein Leistungsspektrum des PPG-Signals zu erzeugen;

Schätzen (206) einer Grundfrequenz f1 als eine Frequenzkomponente mit der höchsten Größe in dem Leistungsspektrum des PPG-Signals über den einen oder die mehreren Hardwareprozessoren;

Berechnen (208) von Oberwellen der Grundfrequenz als gleich eines Vielfachen der Grundfrequenz für 12 Iterationen, um 12 Oberwellen zu erzeugen;

Berechnen der Leistung jeder der 12 Oberwellen;

Bestimmen (210) einer Mehrzahl von Schlüsselunterscheidungsmerkmalen aus der berechneten Leistung jeder der 12 Oberwellen; und

Berechnen (212) einer Wohlbefindensmetrik basierend auf der Mehrzahl von Schlüsselunterscheidungsmerkmalen, wobei die Wohlbefindensmetrik einen Gesundheitszustand des Benutzers darstellt, wobei die Wohlbefindensmetrik verwendet wird, um eine Verbesserung des Gesundheitszustands des Benutzers über einen Zeitraum zu bestimmen, durch:

Berechnen einer ersten Wohlbefindensmetrik zu einem ersten Zeitpunkt T1 über den einen oder die mehreren Hardwareprozessoren;

Berechnen einer zweiten Wohlbefindensmetrik zu einem zweiten Zeitpunkt T2 über den einen oder die mehreren Hardwareprozessoren; und

Vergleichen der ersten Wohlbefindensmetrik und der zweiten Wohlbefindensmetrik, umfassend:

Bestimmen eines ersten Wohlbefindensindex A1 des Benutzers, wobei der erste Wohlbefindensindex ein allgemeines Wohlbefinden des Benutzers darstellt; und

Bestimmen eines zweiten Wohlbefindensindex A2 des Benutzers, wobei der zweite Wohlbefindensindex eine Verbesserung der Gesundheit des Benutzers aufgrund einer oder mehrerer physischer Aktivitäten darstellt, die während der Zeit zwischen T1 und T2 durchgeführt werden.

2. System (100) nach Anspruch 1, wobei das System die Mehrzahl von Schlüsselunterscheidungsmerkmalen durch unabhängiges Berechnen einer Trennungsfähigkeit der Leistung jeder der Oberwellen berechnet.

3. System nach Anspruch 1, wobei das System den ersten Wohlbefindensindex A1 bestimmt als:

$$A_1 = \frac{[\propto(T1) - \propto\_(T2)]}{\propto(T1)}$$

wobei $\alpha$ = die Summe der Leistungen der 5. bis 12. Oberwellen aus den 12 Iterationen, T1 der erste Zeitpunkt ist und T2 der zweite Zeitpunkt ist.

**4.** System nach Anspruch 1, wobei das System den zweiten Wohlbefindensindex A2 bestimmt als:

$$\underline{A_2 = \frac{[\propto_{(T1)} - \propto\_{(T2)}]}{\propto (T1)}}$$

wobei $\alpha$ = die Summe der Leistungen der 7., 9. und 10. Oberwellen aus den 12 Iterationen, T1 der erste Zeitpunkt ist und T2 der zweite Zeitpunkt ist.

**5.** System nach Anspruch 1, wobei das System einen dritten Wohlbefindensindex A3 bestimmt als:

$$A3 = \frac{\sum_{i=5}^{12} P_i}{\sum_{i=1}^{12} P_i}$$

wobei P die Leistung der i-ten Oberwellen ist.

**6.** System nach Anspruch 1, wobei das System einen vierten Wohlbefindensindex A4 bestimmt als:

$$A4 = \frac{\sum_{i=7,9,10} P_i}{\sum_{i=1}^{12} P_i}$$

wobei P die Leistung der i-ten Oberwellen ist.

**7.** Computerprogrammprodukt, umfassend ein nichtflüchtiges computerlesbares Medium mit darin verkörperten computerlesbaren Anweisungen, wobei die computerlesbaren Anweisungen, wenn sie ausgeführt werden, veranlassen, eine Schätzung des Wohlbefindens eines Benutzers durchzuführen durch:

Erfassen eines Photoplethysmogramm-PPG-Signals von dem Benutzer über einen Zeitraum über einen oder mehrere Hardwareprozessoren;
Durchführen einer spektralen Leistungsanalyse des PPG-Signals unter Verwendung von schnellen Fourier-Transformationen (Fast Fourier Transforms) FFT, um ein Leistungsspektrum des PPG-Signals über den einen oder die mehreren Hardwareprozessoren zu erzeugen;
Schätzen einer Grundfrequenz f1 als eine Frequenzkomponente mit der höchsten Größe in dem Leistungsspektrum des PPG-Signals über den einen oder die mehreren Hardwareprozessoren;
Berechnen von Oberwellen der Grundfrequenz als gleich einem Vielfachen der Grundfrequenz für 12 Iterationen, um 12 Oberwellen zu erzeugen, über den einen oder die mehreren Hardwareprozessoren;

Berechnen der Leistung jeder der 12 Oberwellen über den einen oder die mehreren Hardwareprozessoren;
Bestimmen einer Mehrzahl von Schlüsselunterscheidungsmerkmalen aus der berechneten Leistung jeder der 12 Oberwellen über den einen oder die mehreren Hardwareprozessoren; und
Berechnen einer Wohlbefindensmetrik basierend auf der Mehrzahl von Schlüsselunterscheidungsmerkmalen über den einen oder die mehreren Hardwareprozessoren, wobei die Wohlbefindensmetrik einen Gesundheitszustand des Benutzers darstellt, wobei die Wohlbefindensmetrik verwendet wird, um eine Verbesserung des Gesundheitszustands des Benutzers über einen Zeitraum zu bestimmen, umfassend:

Berechnen einer ersten Wohlbefindensmetrik zu einem ersten Zeitpunkt T1 über den einen oder die mehreren Hardwareprozessoren;
Berechnen einer zweiten Wohlbefindensmetrik zu einem zweiten Zeitpunkt T2 über den einen oder die mehreren Hardwareprozessoren; und
Vergleichen der ersten Wohlbefindensmetrik und der zweiten Wohlbefindensmetrik, umfassend:

Bestimmen eines ersten Wohlbefindensindex A1 des Benutzers, wobei der erste Wohlbefindensindex ein allgemeines Wohlbefinden des Benutzers darstellt; und
Bestimmen eines zweiten Wohlbefindensindex A2 des Benutzers, wobei der zweite Wohlbefindensindex eine Verbesserung der Gesundheit des Benutzers aufgrund einer oder mehrerer physischer Aktivitäten darstellt, die während der Zeit zwischen T1 und T2 durchgeführt werden.

**Revendications**

**1.** Système (100) d'estimation du bien-être d'un utilisateur, comprenant :

un ou plusieurs processeurs matériels (104) ;
une ou plusieurs interfaces de communication (106) ; et
une ou plusieurs mémoires (102) stockant une pluralité d'instructions, dans lequel la pluralité d'instructions, lorsqu'elles sont exécutées, amènent les un ou plusieurs processeurs matériels à :

collecter (202) un signal PPG de photopléthysmogramme provenant de l'utilisateur, sur une période de temps ;

effectuer (204) une analyse spectrale de puissance du signal PPG en utilisant une transformée de Fourier rapide, FFT, pour générer un spectre de puissance du signal PPG ;

estimer (206) une fréquence fondamentale f1 en tant que composante de fréquence avec la plus grande amplitude dans le spectre de puissance du signal PPG, via les un ou plusieurs processeurs matériels ;

calculer (208) des harmoniques de la fréquence fondamentale égales à des multiples de la fréquence fondamentale pour 12 itérations pour générer 12 harmoniques ;

calculer la puissance de chacune des 12 harmoniques ;

déterminer (210) une pluralité de caractéristiques distinctives clés à partir de la puissance calculée de chacune des 12 harmoniques ; et

calculer (212) une métrique de bien-être sur la base de la pluralité de caractéristiques distinctives clés, dans lequel la métrique de bien-être représente un état de santé de l'utilisateur, dans lequel la métrique de bien-être est utilisée pour déterminer une amélioration de l'état de santé de l'utilisateur sur une période de temps, en :

calculant une première métrique de bien-être à une première instance de temps T1, via les un ou plusieurs processeurs matériels ;

calculant une seconde métrique de bien-être à une seconde instance de temps T2, via les un ou plusieurs processeurs matériels ; et en

comparant la première métrique de bien-être et la seconde métrique de bien-être, comprenant les étapes menant à :

déterminer un premier indice de bien-être A1 de l'utilisateur, dans lequel le premier indice de bien-être représente un bien-être général de l'utilisateur ; et

déterminer un deuxième indice de bien-être A2 de l'utilisateur, dans lequel le deuxième indice de bien-être représente une amélioration de la santé de l'utilisateur due à une ou plusieurs activités physiques effectuées pendant le temps entre T1 et T2.

2. Système (100) selon la revendication 1, dans lequel le système calcule la pluralité de caractéristiques distinctives clés en calculant une capacité de séparation de la puissance de chacune des harmoniques indépendamment.

3. Système selon la revendication 1, dans lequel le système détermine le premier indice de bien-être A1 comme :

$$A_1 = \frac{[\alpha(T1) - \alpha\_(T2)]}{\alpha(T1)}$$

où, $\alpha$ = la somme des puissances des 5e à 12e harmoniques parmi les 12 itérations, T1 est la première instance de temps, et T2 est la seconde instance de temps.

4. Système selon la revendication 1, dans lequel le système détermine le deuxième indice de bien-être A2 comme :

$$A_2 = \frac{[\alpha(T1) - \alpha\_(T2)]}{\alpha(T1)}$$

où, $\alpha$ = la somme des puissances des 7e, 9e et 10e harmoniques parmi les 12 itérations, T1 est la première instance de temps, et T2 est la seconde instance de temps.

5. Système selon la revendication 1, dans lequel le système détermine un troisième indice de bien-être A3 comme :

$$A3 = \frac{\sum_{i=5}^{12} P_i}{\sum_{i=1}^{12} P_i}$$

où P est la puissance du ie harmonique.

6. Système selon la revendication 1, dans lequel le système détermine un quatrième indice de bien-être A4 comme :

$$A4 = \frac{\sum_{i=7,9,10} P_i}{\sum_{i=1}^{12} P_i}$$

où P est la puissance du ie harmonique.

7. Produit de programme informatique comprenant un support lisible par ordinateur non transitoire ayant des instructions lisibles par ordinateur incorporées dans celui-ci, dans lequel les instructions lisibles par ordinateur, lorsqu'elles sont exécutées, amènent à effectuer une estimation du bien-être d'un utilisateur, par :

la collecte d'un signal PPG de photopléthysmogramme provenant de l'utilisateur, sur une période de temps, via un ou plusieurs processeurs matériels ;

l'exécution d'une analyse spectrale de puissance du signal PPG en utilisant une transformée de Fourier rapide, FFT, pour générer un spectre de puissance du signal PPG, via les un ou plusieurs processeurs matériels ;

l'estimation d'une fréquence fondamentale f1 en tant que composante de fréquence avec la plus grande amplitude dans le spectre de puissance du signal PPG, via les un ou plusieurs processeurs matériels ;

le calcul d'harmoniques de la fréquence fondamentale égales à des multiples de la fréquence fondamentale pour 12 itérations pour générer 12 harmoniques, via les un ou plusieurs processeurs matériels ;

le calcul de la puissance de chacune des 12 harmoniques, via les un ou plusieurs processeurs matériels ;

la détermination d'une pluralité de caractéristiques distinctives clés à partir de la puissance calculée de chacune des 12 harmoniques, via les un ou plusieurs processeurs matériels ; et

le calcul d'une métrique de bien-être sur la base de la pluralité de caractéristiques distinctives clés, via les un ou plusieurs processeurs matériels, dans lequel la métrique de bien-être représente un état de santé de l'utilisateur, dans lequel la métrique de bien-être est utilisée pour déterminer une amélioration de l'état de santé de l'utilisateur sur une période de temps, comprenant :

le calcul d'une première métrique de bien-être à une première instance de temps T1, via les un ou plusieurs processeurs matériels ;

le calcul d'une seconde métrique de bien-être à une seconde instance de temps T2, via les un ou plusieurs processeurs matériels ; et

la comparaison de la première métrique de bien-être et de la seconde métrique de bien-être, comprenant :

la détermination d'un premier indice de bien-être A1 de l'utilisateur, dans lequel le premier indice de bien-être représente un bien-être général de l'utilisateur ; et

la détermination d'un deuxième indice de bien-être A2 de l'utilisateur, dans lequel le deuxième indice de bien-être représente une amélioration de la santé de l'utilisateur due à une ou plusieurs activités physiques effectuées pendant le temps entre T1 et T2.

FIG. 1

```
┌──────────────────────────────┐  202
│                              │
│  Collecting PPG signals from a user │
│                              │
└──────────────────────────────┘
              │
              ▼
┌──────────────────────────────┐  204
│ Performing power spectral analysis of │
│  the PPG signal to generate a power │
│    spectrum of the PPG signal │
└──────────────────────────────┘
              │
              ▼
┌──────────────────────────────┐  206
│  Estimating a fundamental frequency │
│       of the PPG signal │
└──────────────────────────────┘
              │
              ▼
┌──────────────────────────────┐  208
│ Calculating upto 12th harmonics of the │
│   fundamental frequency i.e. 12 │
│           iterations │
└──────────────────────────────┘
              │
              ▼
┌──────────────────────────────┐  210
│   Determining key distinguishing │
│ features from the calculated power of │
│     each of the harmonics │
└──────────────────────────────┘
              │
              ▼
┌──────────────────────────────┐  212
│ Calculating a wellness metric of the │
│ user, based on the key distinguishing │
│          parameters │
└──────────────────────────────┘
```

200

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20120021098 A